(19) Europäisches Patentamt
European Patent Office
Office européen des brevets

(11) **EP 2 514 379 A1**

(12) **EUROPEAN PATENT APPLICATION**
published in accordance with Art. 153(4) EPC

(43) Date of publication:
**24.10.2012 Bulletin 2012/43**

(51) Int Cl.:
*A61B 18/04* (2006.01)   *A61N 1/04* (2006.01)
*A61B 5/04* (2006.01)   *A61B 19/00* (2006.01)

(21) Application number: **10836945.5**

(22) Date of filing: **27.04.2010**

(86) International application number:
**PCT/CN2010/072266**

(87) International publication number:
**WO 2011/072507 (23.06.2011 Gazette 2011/25)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **15.12.2009 CN 200910311495**

(71) Applicant: **Sichuan Jinjiang Electronic And Technology Co., Ltd.**
**Sichuan 610000 (CN)**

(72) Inventors:
• **LI, Chuwen**
**Chengdu**
**Sichuan 610000 (CN)**

• **LI, Bin**
**Chengdu**
**Sichuan 610000 (CN)**
• **XUE, Fen**
**Chengdu**
**Sichuan 610000 (CN)**

(74) Representative: **Lermer, Christoph et al**
**LangRaible GbR**
**Patent- und Rechtsanwälte**
**Rosenheimer Strasse 139**
**81671 München (DE)**

(54) **METHOD AND APPARATUS FOR LOCATION DETERMINATION OF CARDIAC CATHETER**

(57) The invention discloses a method for positioning an intracardiac catheter, comprising the following steps: A: obtain collected position signals of the intracardiac catheter and reference signals comprising respiration information; B: utilizes a self-adaptation de-noising method to process respiration information signals in A step, and eliminate respiration fluctuations in the collected position signals of the catheter. As influences which the same breath has on the catheters at different positions of the heart are related, the invention utilizes the self-adaptation de-noising principle to introduce the reference signals conprising the respiration information and to offset the respiratory displacement in the positioned catheter in a cardiac chamber, without other distorted signals, which hence can avoid using low-pass filtering, and reduce real-time movement of the catheter while having no any ineffective impact. Due to real-time tracking filtering, there is not any problem of different effects at different parts. Furthermore, systems, positioned by electric and magnetic fields as well as ultrasound, are all applicable.

Figure 1

EP 2 514 379 A1

**Description**

**Technical Field of the Invention**

The invention relates to a method and a device for positioning an intracardiac catheter, in particular to a method and a device for positioning the intracardiac catheter with a respiration compensation technique.

**Technical Background of the Invention**

A 3-D mapping system positioned by an intracardiac catheter is mainly used for an arrhythmia surgery of Radiofrequency Catheter Ablation (RFCA), records 3-D spatial locations of the intracardiac catheter, utilizes the obtained information to establish a 3-D inracardiac anatomy geometric graph, and carries out excitement time and excitement spread mappings, wherein the precision of electrode position of the catheter will directly influence the shape of a model, and guide ablation effects.

Regardless of a system positioned by magnetic field or electric field, the intracardiac catheter will be influenced by breath and heart beats and will be fluctuated. If heartbeat frequency is relatively faster than catheter movement, the problem can be solved by filtering or sampling during particular heartbeats. The respiration influence will hence be a main factor causing electrodes of a catheter to have positioning error. Especially in a system positioned by the electric field, besides the respiration changes the intracardiac catheter's own position, it will also change a passage of excitation current and impedance of blood returned back to a cardiac chamber from the lungs due to the breath in lungs, thus resulting in bigger influence.

There are mainly two kinds of respiration compensations in the prior art:

firstly, low-pass filtering: respiration band is generally 0.1 - 2HZ and overlapped with motion frequency of the catheter. If relatively low cutoff frequency is adopted, movement information of the catheter will be filtered, thus influencing real-time movement of the catheter. If relatively high cutoff frequency is adopted, respiration disturbance can not be filtered completely. For example, if the low-pass filtering of the cutoff frequency of 0.25HZ is adopted, respiration influence will cause the catheter position to be fluctuated by 1 - 2cm; and

secondly, a respiration compensation method in United States Patent7,263,397: the method is not suitable for a system positioned by magnetic field, and furthermore, it is very complicated. As for the method, not only that an excitation method of 3-axis electric field is changed into a switch method of a multi-pole electric field, but also that when respiration compensation coefficient is calculated, the catheter is required to have been stable for 10 seconds before data are collected. As different parts in cardiac chambers have different phases and amplitudes, data at different parts, which will have been collected for 10 seconds, should be calculated to guarantee effects. When compensation effects at different parts have bed effects, coefficients should be calculated again.

**Summary of the Invention**

The invention aims at providing a respiration compensation technique based on self-adaptation filtering, which can accurately position an intracardiac catheter.

The technical proposal of the invention is as follows:

1. A method for positioning an intracardiac catheter, comprising the following steps:

A, obtain collected position signals of the intracardiac catheter and reference signals comprising respiration information; and

B. utilize a self-adaptation de-noising method to process the signals of the respiration information in A step, and eliminate respiration fluctuations in the collected position signals of the intracardiac catheter.

A device for positioning an intracardiac catheter comprises input of collected position signals of the intracardiac catheter, and a data processing unit, wherein it also comprises input of reference signals comprising respiration information. The data processing unit, after the collected position signals and reference signals are input into the data-processing unit, should process the respiration information in the reference signals through a self-adaptation de-noising method, thus eliminating respiration fluctuations in the collected position signals of the catheter.

The additional technical proposal of the invention is as follows:

Preferably, the introduced reference signals comprise the respiration information. The reference signals, adopting one or several methods of electric and magnetic fields as well as ultrasound, are obtained at body surface or in some part of the heart.

Preferably, before the reference signals are processed with the self-adaptation de-noising method, DC displacement signals in the reference signals, which comprise their own information on fixed positions, are firstly filtered, and then the signals only comprising the information related to respiration are obtained.

Preferably, during the self-adaptation de-noising process, weight coefficient W1 is utilized to track respiration movement of a positioned catheter, and weight coefficient W2 is utilized to track the displacement of the positioned catheter, wherein a rejustment method of the weight coefficient W1 or the weight coefficient W2 is carried out through the adoption of an LMS (Least Mean Square) self-adaptation algorithm with the variable steps.

Preferably, during the self-adaptation de-noising process, the weight coefficient W1 is utilized to track the respiration movement of the positioned catheter, and the weight coefficient W2 is utilized to track the displacement of the positioned catheter, wherein the rejustment method of the weight coefficient W1 or the weight coefficient W2 is carried out through the adoption of the LMS self-adaptation algorithm with the variable steps.

The invention has the following advantages: as influences which the same breath has on the catheters at different positions of the heart are related, the invention utilizes a self-adaptation de-noising principle to introduce the reference signals comprising the respiration information and to offset the respiratory displacement in the positioned catheter in cardiac chambers, without other distorted signals, which hence can avoid using low-pass filter and reduce real-time movement of the catheter while having no any ineffective impact. Due to real-time tracking filtering, there is not problem of different effects at different parts. Furthermore, systems, positioned by electric and magnetic fields as well as ultrasound, are all applicable.

**Drawings of the Invention**

The invention is described through examples and accompanying drawings, wherein:

> Fig. 1 is a principle block diagram according to embodiments of the invention.
> Fig. 2 is a schematic diagram of a catheter positioned in a cardiac chamber.
> Fig. 3 is an actual design sketch of respiration compensation.
> Fig. 4 is a flow chart of a self-adaptation compensation algorithm.

**Embodiments of the invention**

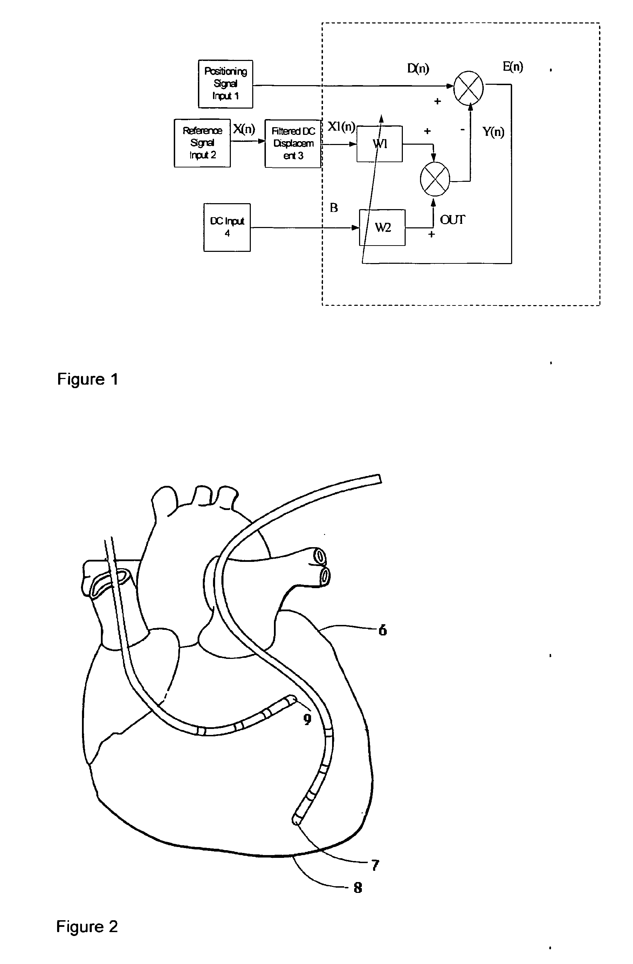

As shown in Figs.1 and 2 of the invention, a method for positioning an intracardiac catheter, comprising the following steps:

> A. obtain collected position signals of the intracardiac catheter and reference signals comprising respiration information; and
> B. utilize a self-adaptation de-noising method to process the signals of the respiration information in A step, and eliminate respiration fluctuations in the collected position signals of the intracardiac catheter.

The above position signals and the reference signals comprising respiration information are obtained through the following methods:

As shown in Fig. 2, position signals are collected in a heart 6 with a catheter 7. Currently, the catheter is positioned at left cardiac chamber 8.The position signals can be obtained through adoption of any one of methods such as electric field, magnetic field and ultrasound. The collected position signals generally comprise positioning information of X, Y and Z directions. The compensation operation shown in Fig. 1 is carried out for the three directions, respectively. Now, signals D(n) in the Y direction are taken as positioning signals to be illustrated, and other directions are the like.

When reference signals are collected with selection of the intracardiac catheter, one catheter 9 positioned in coronary sinus of a heart is adopted to collect the reference signals, because the catheter can not generally be moved during surgery and only fluctuates with breath and heart beats. The heartbeat information comprised in the reference signals does not influence effects of respiration compensation. In additions, the reference signals can also be collected with the selection of electrode on body surface, and only the electrode is required to be attached to chest.

Similarly, the reference signals can also be obtained through adoption of any one of methods such as electric field, magnetic field and ultrasound. Generally, the signals also comprise the information on the X, Y and Z directions; however, the method shall be the same as the realized method of positioned input signals 1, and any one of the directions can be selected. Now, signals X(n) in the Y direction are taken as positioning input signals 1 to be illustrated, and other directions are the like.

In order to further optimize evaluation effects of the reference signals, before the reference signals are processed with a self-adaptation de-noising method, DC displacement signals in the reference signals, which comprise their own information on fixed positions, are firstly filtered, and then the signals only comprising the information related to respiration are obtained. As shown in Fig. 1, effect of filtered DC displacement 3 indicates that the DC components in signals X (n) shall be filtered, and the reference signals are facilitated to only comprise respiration information, both of which can be realized through high-pass filtering or the adoption of the method that movement average is subtracted.

During the process that DC displacement signals are filtered, as the reference signals comprise relatively less displacement, the process has small calculation quantity and can be easily realized by the adoption of the method that movement average is subtracted. The selection of mobile time window shall at least comprise one respiration period. Considering the respiration of frequency of 0.1 HZ, at least 10S data shall be selected and taken as the mobile time window. When sampling period is 16mS, 625 points shall be required, in which:

$$X1(n) = X(n) - (X(n) + X(n-1) + X(n-2)...+X(n-624)) /625$$

In the example, the effect of introduced DC input 4 indicates that low-frequency reference related to displacement signals of a catheter is introduced. The low-frequency reference is utilized to track the displacement of the positioned catheter. Fixed DC input B=1 can be selected.

The above signals are carried out with the self-adaptation de-noising method and the virtual box portion in the figure is the self-adaptation de-noising portion. The self-adaptation filter is essentially a special Wiener filter which can automatically adjust its own parameters. When the filter is designed, the statistics knowledge on input signals and noise are not required to be grasped in advance. The filter can gradually "understand" or estimate the required statistic properties during its own operation process, on which the filter bases to automatically adjust its own parameters and achieve the best filtering effect. Once the statistic properties of the input signals are changed and the filter can track these changes, the filter will automatically adjust the parameters and facilitates its properties to be the best ones again.

Two kinds of self-adaptation algorithms are illustrated through the following examples:

The first kind: an LMS algorithm with variable steps is adopted:

As for the invention, weight coefficient W1 is utilized to track the respiration movement of a positioned catheter, and weight coefficient W2 is utilized to track the displacement of the positioned catheter.

The concrete adopted iterative formulas are as follows:

$$Y(n)=X1(n)W1(n)+BW2(n) \qquad (1)$$

$$E(n)=D(n)-Y(n) \qquad (2)$$

$$W1(n+1)=W1(n)+ \mu1(n)E(n)X1(n) \qquad (3)$$

$$\mu1(n+1) = \alpha1\mu1(n) +\gamma1P1(n) \qquad (4)$$

Furthermore:

$$\text{If } \mu1(n+1) > \mu1max$$

$$\mu1(n+1) =\mu1max$$

$$\text{if } \mu1(n+1) < \mu1min$$

$$\mu1(n+1) =\mu1min$$

$$\text{else}\mu1(n+1) =\mu1(n+1) \qquad (5)$$

In formula (4): $0 < \alpha1 < 1$, $\gamma1 > 0$; in formula (5): the upper bound $\mu1max$ and the lower bound $\mu1min$ of step are set. The $\mu1max$ defines the maximum possible convergence rate while the $\mu1min$ guarantees relatively small steady-state error. The step of algorithm is actually controlled by $\alpha1$ and $\gamma1$. $\alpha1$ is a genetic factor of the step and mainly determines the values of the step when the algorithm is converged. When the algorithm depth is converged, $\gamma1P1(n) \rightarrow 0$. Basically, the step is reduced according to index $\alpha1n$ until $\mu1(n) \rightarrow \mu1min$. $\gamma1$ determines the influenced degree of the step by the energy $P1(n)$ and mainly controls starting and tracking speeds of the algorithm.

$$P1(n) = \beta1P1(n-1) + (1-\beta1)E(n)E(n-1) \quad (6)$$

In the formula (6): $0 < \beta1 < 1$.

$$W2(n+1) = W2(n) + \mu2(n)E(n)B \quad (7)$$

$$\mu2(n+1) = \alpha2\mu2(n) + \gamma2P2(n) \quad (8)$$

Furthermore,

$$\text{If } \mu2(n+1) > \mu2max$$

$$\mu2(n+1) = \mu2max$$

$$\text{if } \mu2(n+1) < \mu2min$$

$$\mu2(n+1) = \mu2min$$

$$\text{else} \mu2(n+1) = \mu2(n+1) \quad (9)$$

$$P2(n) = \beta2P2(n-1) + (1-\beta2)E(n)E(n-1) \quad (10)$$

$$OUT(n) = BW2(n) \quad (11)$$

In formulas (8), (9) and (10), the definitions of $\alpha2$, $\gamma2$, $\mu2max$, $\mu2min$ and $\beta2$ are similar to those of the above-mentioned $\alpha1$, $\gamma1$, $\mu1max$, $\mu1min$ and $\beta1$.

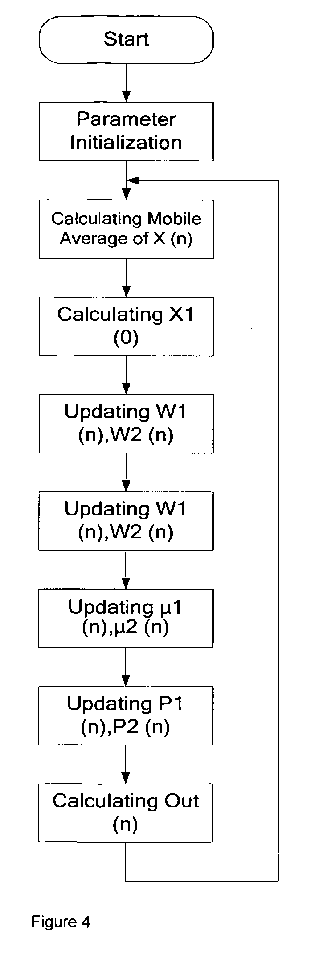

Fig. 4 is a software flow chart of the algorithm which is concrete demonstration of the above formulas. The algorithm is realized with adoption of iterative and recursive methods, thus having the advantages of small quantity of calculation, great real time and less occupying resources. The algorithm can be realized with the adoption of an upper layer computer or a lower layer DSP, a single chip microcomputer, a programmable chip and other means.

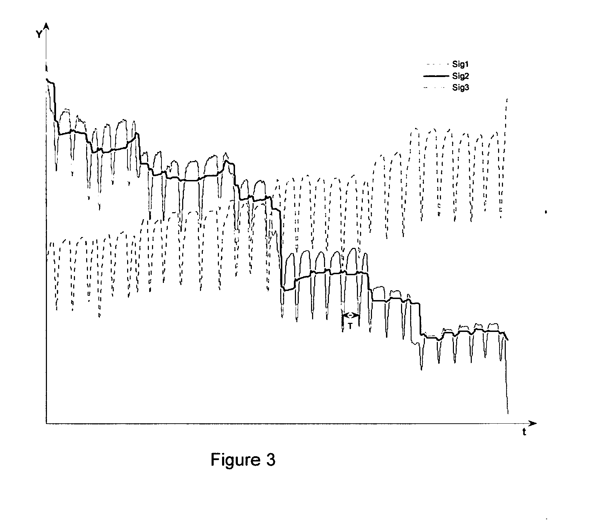

Fig. 3 is an actual design sketch after use of the algorithm, wherein Sig1 is mobile trajectory in Y direction of positioned catheter comprising respiration movement. Sig2 is trajectory in Y direction of a reference catheter comprising respiration movement. Sig3 is mobile trajectory in Y direction of a positioned catheter after respiration compensation. T is one respiration cycle. Visible signal Sig3 not only filters the respiration influence but also does not influence tracking of real-time movement of a catheter.

The second kind: LMS Self-Adaptation De-Noising Algorithm.

Weight coefficient W1 is utilized to track the respiration movement of a positioned catheter, and weight coefficient W2 is utilized to track the displacement of the positioned catheter.

W1 rejustment herein indicates specifically an M-th order filter of which the weight coefficient is W1i.

The concrete adopted iterative formulas are as follows:

$$Y(n)=\sum_{i=0}^{M-1} W1i(n)X1(n\text{-}i)+BW2(n) \qquad 0 \le i \le M\text{-}1 \qquad (1)$$

$$E(n)=D(n)\text{-}Y(n) \qquad (2)$$

$$W1i_{(n+1)}= W1i_{(n)}+ 2\mu 1E(n)X1(n\text{-}i) \qquad 0 \le i \le M\text{-}1 \qquad (3)$$

$$W2(n+1)=W2(n)+ 2\mu 2E(n)B \qquad (4)$$

$\mu 1$ is the step of tracking respiration.

Step factor $\mu 1$, order number of M of a filter and the power of input signals X1are all related. To enable the convergence of the system, in the case of the same input signals, the value of $\mu 1$ should be inversely proportional to the order number of the filter, and different steps should be adopted based on different order number of the filter, so as to ensure the best signal processing results. When M is fixed, $\mu 1$ is the only parameter affecting the convergence speed of LMS algorithm, and it will be changed with the change of input signal power. $\mu 1$ value can not be too large, because when the $\mu 1$ value is too large, the relatively large gradient noise will be introduced during the self-adaptive process, and the transition process will appear oscillations and can not be converged. If $\mu 1$ value is too small, although the gradient noise is reduced, the convergence speed will be relatively slow. Therefore $\mu 1$ value shall have compromise consideration.

The determination of M of order number of a filter: when M is low, forecasted parameters will be less and error will be relatively large, especially when the respiration is irregular. Meanwhile, if the phase between the respiration information of reference signals and positioning signals is relatively highly different, convergence time will be longer.

When M is high, output can not reflect timely the rapid change of the input; meanwhile, considerable errors may be introduced.

$\mu 2$ is step tracking displacement of a positioned catheter.. When the $\mu 2$ value chosen is too large, oscillations may appear during the transition and the $\mu 2$ value can not be converged. If $\mu 2$ value selected is too less, the tracking speed of the positioned catheter may be influenced; therefore, the $\mu 2$ value should have compromise consideration. If it is necessary, according to different E(n), different $\mu 2$ values can be selected based on different fragments.

As for the abovementioned two algorithms, LMS with variable steps has advantages of rapid convergence and less error.

A device running the above embodiments is consistent with the traditional inracardiac catheter, comprising a position-colleting catheter and a reference-signal collecting catheter or a body-surface electrode, and a probe amounted on the catheter. Data collected pass through a data-collecting device, enters a computer or DSP, a single-chip microcontroller, a programmable chip and other means, and are processed in a data processing means according to the above steps.

All characteristics disclosed in the specification, all the disclosed methods or steps of processes can be combined in any manner, except mutually exclusive characteristics and / or steps.

Any one of the characteristics disclosed in this specification (including any additional claim, abstract and accompany drawings), unless particularly described, all can be replaced with other equivalents or alternative characteristics with similar purposes. That is, unless particularly described, each feature is just one example among a series of equivalents or similar features.

The invention is not limited to the aforementioned embodiments. The invention extends to any disclosed new feature or any new combination thereof, as well as any one of disclosed new methods, process steps or any new combination thereof in this specification.

**Claims**

1. A method for positioning an intracardiac catheter, comprising the following steps:

    A, obtain collected position signals of the intracardiac catheter and reference signals comprising respiration information; and
    B, utilize a self-adaptation de-noising method in A step to process the signals of the respiration information, and eliminate respiration fluctuations in the collected position signals of the intracardiac catheter.

2. The method for positioning the intracardiac catheter of Claim 1, wherein the introduced reference signals comprise the respiration information. The reference signals, adopting one or several the methods of the electric and magnetic fields as well as ultrasound, are obtained at body surface or some part of the heart.

3. The method for positioning the intracardiac catheter of Claim 1 or 2, wherein before the reference signals are processed with a self-adaptation de-noising method, DC displacement signals, which are in the reference signals and comprise their own information on fixed positions, are firstly filtered, and then the signals only comprising the information related to respiration are obtained.

4. The method for positioning the intracardiac catheter of Claim 1 or 2, wherein during the self-adaptation de-noising process, weight coefficient W1 is utilized to track respiration movement of the positioned catheter; weight coefficient W2 is utilized to track the displacement of the positioned catheter, wherein a rejustment method of the weight coefficient W1 or the weight coefficient W2 is carried out through the adoption of an LMS self-adaptation algorithm with variable steps.

5. The method for positioning the intracardiac catheter of Claim 3, wherein during the self-adaptation de-noising process, the weight coefficient W1 is utilized to track the respiration movement of the positioned catheter; the weight coefficient W2 is utilized to track the displacement of the positioned catheter, wherein the rejustment method of the weight coefficient W1 or the weight coefficient W2 is carried out through the adoption of the LMS self-adaptation algorithm with the variable steps.

6. The method for positioning the intracardiac catheter of Claim 3, wherein during the self-adaptation de-noising process, the weight coefficient W1 is utilized to track the respiration movement of the positioned catheter; the weight coefficient W2 is utilized to track the displacement of the positioned catheter, wherein the rejustment method of the weight coefficient W1 or the weight coefficient W2 is carried out through the adoption of the LMS self-adaptation algorithm with the variable steps.

7. A device for positioning an intracardiac catheter, comprising input of collected position signals of an intracardiac catheter, and a data processing unit, wherein the device also comprises input of reference signals of respiration information; the data processing unit, after collected position signals and reference signals are input into the data processing unit, processes the respiration information in the reference signals with a self-adaptation de-noising method, thus eliminating respiration fluctuations in the collected position signals of the catheter.

Figure 1

Figure 2

Figure 3

Figure 4

# INTERNATIONAL SEARCH REPORT

| International application No. |
|---|
| PCT/CN2010/072266 |

**A. CLASSIFICATION OF SUBJECT MATTER**

See extra sheet

According to International Patent Classification (IPC) or to both national classification and IPC

**B. FIELDS SEARCHED**

Minimum documentation searched (classification system followed by classification symbols)

IPC: A61B A61N

Documentation searched other than minimum documentation to the extent that such documents are included in the fields searched

Electronic data base consulted during the international search (name of data base and, where practicable, search terms used)

CNPAT WPI EPODOC CNKI   catheter+, canal+, conduit+, orient+, local+, posit+, cardio+, heart+, breath+, respire+, vent+, adapt+, LMS+, RFCA

**C. DOCUMENTS CONSIDERED TO BE RELEVANT**

| Category* | Citation of document, with indication, where appropriate, of the relevant passages | Relevant to claim No. |
|---|---|---|
| X | CN100546540 C (SHANGHAI HONGTONG INDUSTRY CO), 07 Oct.2009 (07.10.2009), page 2 line 4 to page 4 line 23, page 7 line 3 to page 8 line 6 in the specification, figures 1-3. | 1-7 |
| X | CN200970234 Y (SHANGHAI HONGTONG INDUSTRY CO), 07 Nov. 2007 (07.11.2007), page 2 line 4 to page 4 line 19, page 6 line 27 to page 8 line 2 in the specification, figures 1-3. | 1-7 |
| X | US7263397 B2 (ST JUDE MEDICAL ATRIAL FIBRILL), 28 Aug.2007 (28.08.2007), column 1 line 54 to column 3 line 5, column 6 line 30 to column 7 line 14, column 9 line 15 to column 10 line 10 in the specification, figures 1,6,8a-8d. | 1-3,7 |

☒ Further documents are listed in the continuation of Box C.　　　☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim (S) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&"document member of the same patent family |

| Date of the actual completion of the international search | Date of mailing of the international search report |
|---|---|
| 26 Aug.2010 (26.08.2010) | **16 Sep. 2010 (16.09.2010)** |

| Name and mailing address of the ISA/CN<br>The State Intellectual Property Office, the P.R.China<br>6 Xitucheng Rd., Jimen Bridge, Haidian District, Beijing, China 100088<br>Facsimile No. 86-10-62019451 | Authorized officer<br><br>　　　　PANG,Qingfan<br><br>Telephone No. (86-10)62085624 |
|---|---|

Form PCT/ISA /210 (second sheet) (July 2009)

## INTERNATIONAL SEARCH REPORT

| | International application No. |
| --- | --- |
| | PCT/CN2010/072266 |

C (Continuation).     DOCUMENTS CONSIDERED TO BE RELEVANT

| Category* | Citation of document, with indication, where appropriate, of the relevant passages | Relevant to claim No. |
| --- | --- | --- |
| A | EP0775466 A2 (MEDTRONIC INC), 28 May 1997 (28.05.1997), the whole document. | 1-7 |
| A | US5433198 A (DESAI J M), 18 Jul.1995 (18.07.1995), the whole document. | 1-7 |
| A | CN1415275 A (ZHAO, Yaode et al), 07 May 2003 (07.05.2003), the whole document. | 1-7 |

Form PCT/ISA /210 (continuation of second sheet ) (July 2009)

EP 2 514 379 A1

## INTERNATIONAL SEARCH REPORT
Information on patent family members

International application No.

PCT/CN2010/072266

| Patent Documents referred in the Report | Publication Date | Patent Family | Publication Date |
|---|---|---|---|
| CN100546540 C | 07.10.2009 | CN101147676 A | 26.03.2008 |
| CN200970234 Y | 07.11.2007 | None | |
| US7263397 B2 | 28.08.2007 | US2004254437 A1 | 16.12.2004 |
| | | US2003120318 A | 26.06.2003 |
| | | US7187973 B | 06.03.2007 |
| | | US2005203394 A | 15.09.2005 |
| | | US7670297 B | 02.03.2010 |
| EP0775466 A2 | 28.05.1997 | AU7184296 A | 29.05.1997 |
| | | JP9168519 A | 30.06.1997 |
| | | CA2189399 A | 23.05.1997 |
| | | US5697377 A | 16.12.1997 |
| | | US5983126 A | 09.11.1999 |
| | | AU714372 B2 | 23.12.1999 |
| | | EP0775466 B1 | 22.10.2003 |
| | | DE69630432 E | 27.11.2003 |
| | | JP3955346 B2 | 08.08.2007 |
| | | CA2189399 C | 07.10.2008 |
| | | DE69630432 T2 | 06.05.2004 |
| | | AU714372 B2 | 23.12.1999 |
| US5433198 A | 18.07.1995 | WO9632897 A1 | 24.10.1996 |
| | | AU2360695 A | 07.11.1996 |
| | | EP0830091 A1 | 25.03.1998 |
| | | AU697414 B | 08.10.1998 |
| | | EP0830091 B1 | 29.10.2003 |
| | | EP1419742 A2 | 19.05.2004 |
| | | JP3712265 B2 | 02.11.2005 |
| | | US2007161915 A1 | 12.07.2007 |
| | | US5657755 A | 19.08.1997 |
| | | US2002010392 A | 24.01.2002 |
| | | US6522905 B | 18.02.2003 |
| | | US2003181819 A | 25.09.2003 |
| | | US2005148892 A | 07.07.2005 |
| | | US2010204598 A1 | 12.08.2010 |
| CN1415275 A | 07.05.2003 | None | |

Form PCT/ISA /210 (patent family annex) (July 2009)

# INTERNATIONAL SEARCH REPORT

International application No.

PCT/CN2010/072266

CLASSIFICATION OF SUBJECT MATTER

A61B 18/04 (2006.01) i
A61N 1/04 (2006.01) i
A61B 5/04 (2006.01) i

A61B 19/00 (2006.01) i

Form PCT/ISA /210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7263397 B